# EUROPEAN PATENT SPECIFICATION

(11) **EP 0 650 496 B1**
(45) Date of publication and mention of the grant of the patent: **16.09.1998**
(21) Application number: 93913939.0
(22) Date of filing: 17.05.1993
(51) Int. Cl.: C07J 5/00, C07J 31/00, C07J 9/00, A61K 31/57, A61K 31/575

(54) **ANTI-INFLAMMATORY COMPOUNDS FOR OPHTHALMIC USE**
Entzündungshemmende Verbindungen zur ophthalmischer Verwendung
COMPOSES ANTI-INFLAMMATOIRES DESTINES A UN USAGE OPHTALMIQUE

(43) Date of publication of application: 03.05.1995
(73) Proprietor: ALCON LABORATORIES, INC., Fort Worth Texas 76134-2099 (US)
(72) Inventor: BOLTRALIK, John, J., Fort Worth, TX 76133 (US)
(74) Representative: Keller, Günter, Dr.
(86) International application number: US9304671
(87) International publication number: WO9426769

(56) References cited:
- EP-A- 0 004 765
- EP-A- 0 320 253
- CHEMICAL ABSTRACTS, vol. 113, no. 15, 08 October 1990, Columbus, OH (US); M. MIKUCHI et al., p. 693, AN 132583d
- CHEMICAL & PHARMACEUTICAL BULLETIN, vol. 38, no. 3, March 1990, Tokyo (JP); M. MITSUKUCHI et al., pp. 786-789
- JOURNAL OF THE CHEMICAL SOCIETY, no. 8, August 1981, Oxford (GB); J. CAIRNS et al., pp. 2306-2316

## Description

The present invention relates to novel derivatives of known anti-inflammatory steroids, processes useful for their preparation, and use of these novel derivatives in the treatment of ophthalmic inflammatory disorders. More particularly, this invention relates to the treatment of ophthalmic inflammatory disorders with novel derivatives of known anti-inflammatory steroids, wherein the novel derivatives do not cause any significant increase in intraocular pressure.

Anti-inflammatory steroids, such as hydrocortisone, prednisolone, dexamethasone, and fluorometholone, are very useful in controlling a wide range of ophthalmic inflammatory conditions. This usefulness may be somewhat negated due to a steroid-induced side effect associated with the chronic use of these compounds. This side effect may be manifested by a rise in intraocular pressure (IOP) in steroid-sensitive patients (steroid-responders), an increase in IOP in glaucoma sensitive patients, or an exacerbation of IOP in patients suffering from frank primary open angle glaucoma. Further discussion of this side effect is presented in an article by Phillips, et al., The Lancet, 767-768 (April 7, 1984).

The above-described manifestations can generally be tolerated in most patients over a relatively short treatment period (i.e., four to six weeks, or less); however, the increase in IOP caused by these compounds is generally unacceptable over extended periods of treatment (i.e., one to twelve months, or more), especially in individuals subject to chronic eye inflammation. The increased intraocular pressure associated with the short term use of these compounds may also be unacceptable in certain patients, such as patients already suffering from an elevated IOP (e.g., glaucoma patients). A need therefore exists for an effective means of treating inflamed ocular tissue without risk of elevating IOP. The present invention is directed to satisfying this need.

EP-A 320 253, Chem. Abstr. 113, 132583d (1990) and Chem. Pharm. Bull. 38 (3), 786 (1990) disclose some steroidal compounds having anti-inflammatory activity. None of these documents is directed to ophthalmic compositions. JCS Perkin I 2306 (1981) discloses 16α,17α,21-trimethyl steroids possessing anti-inflammatory activity. One side product of certain 16α,17α,21-trimethyl steroids is reported to be a steroidal 21-methylether. This document does not disclose any ophthalmic compositions either.

### Summary of the Invention

The present invention is directed to novel steroidal C21 ether derivatives, ophthalmic pharmaceutical compositions containing the derivatives, and methods of treating ophthalmic inflammatory disorders with these compositions wherein no significant increase in intraocular pressure results.

It is accepted, from studies of structure-activity relationships in steroid chemistry, that seemingly minor structural modifications in a steroid molecule can either signficantly enhance or adversely affect the pharmacological properties of the compound being studied, presumably by altering the action of the drug at the target cells. These modifications may confer agonistic or antagonistic properties to the steroid molecule. Agonistic drugs are broadly defined as compounds which elicit or activate a biochemical response in situations where the mechanism which elicits the response is undefined. On the other hand, antagonistic drugs are broadly defined as inhibitors of the above-identified selective responses. For example, it has been established that certain side chain modifications affect IOP regulation by depressing or inhibiting the systems responsible for directing the synthesis of proteins which regulate the unwanted IOP side effects. Therefore, if a given steroid is designed to have properties which provide both characteristics of agonism with respect to the retention of anti-inflammatory properties, and antagonism with respect to complete suppression of IOP increase, a true separation of effects is achieved.

The present invention is based on the discovery of novel derivatives of certain known anti-inflammatory steroids, such as dexamethasone and fluocinolone, which confer the desired separation of effects. For example, replacement of the proton of the dexamethasone C21 hydroxyl group with a methyl group to produce a C21 methyl ether results in a compound having the anti-inflammatory properties of dexamethasone, but without elevation of intraocular pressure, contrary to what is normally seen with dexamethasone and other steroids. This holds true even with chronic use (e.g., one month or longer).

### Detailed Description of the Invention

The steroid derivatives which have been found to exhibit the above-discussed separation of effects have the following formulae (I) and (II): 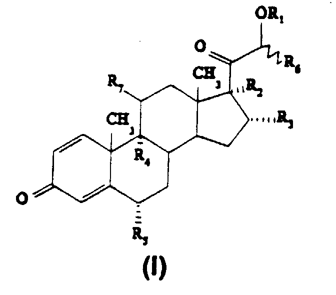 wherein:
- R₁: is selected from substituted or unsubstituted: C₁-C₅ alkyl; C₃-C₆ (ring) and C₁-C₃ (alkyl) cycloalkyl and cycloalkylalkyl; aryl and arylalkyl, such as phenyl and benzyl; and C₂-C₅ alkenyl and alkynyl; wherein the substituent or substituents are selected from: C₁-C₆ alkoxy and alkyl; C₂-C₆ alkoxyalkyl, alkoxyalkoxyalkyl, alkylthioalkyl, hydroxyalkyl, aminoalkyl, nitriloalkyl; C₄-C₈ (beta-carboxyalkoxy)alkyl; and C₂-C₆ alkenoxy and alkynoxy;
- R₂: is selected from hydrogen and substituted or unsubstituted: C₁-C₆ alkyl, alkenyl and alkynyl; acyloxy (-OCOR'; wherein, for example, R' is a C₁-C₆ alkyl); and hydroxyl; wherein the substituent or substituents are selected from: C₁-C₃ alkyl; hydroxyl; and halogen (F, Cl, and Br);
- R₃: is selected from methyl and hydroxyl;
- R₄: is selected from hydrogen, fluoro, and chloro;
- R₅: is selected from hydrogen, methyl, fluoro, and chloro;
- R₆: is selected from hydrogen, methyl, ethyl, and allyl; and
- R₇: is selected from hydroxyl, acetyloxy, propanoyloxy, and butanoyloxy;
and: 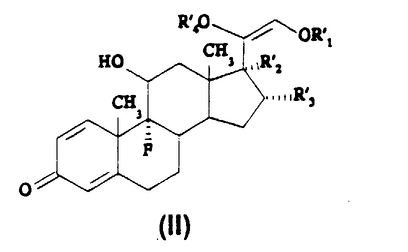 wherein:
- R'₁: is selected from substituted or unsubstituted: C₁-C₅ alkyl, alkenyl, and alkynyl;
- R'₂: is selected from hydrogen and substituted or unsubstituted: C₁-C₆ alkyl, alkenyl, alkynyl; acyloxy; and hydroxyl; wherein the substituent or substituents are selected from: C₁-C₃ alkyl; hydroxyl; and halogen;
- R'₃: is selected from methyl and hydroxyl; and
- R'₄: is selected from C₁-C₅ alkyl, alkenyl, and alkynyl.

These compounds possess anti-inflammatory activity similar to that of known steroids (e.g., dexamethasone), but do not significantly elevate intraocular pressure when applied to inflamed ocular tissue.

The compounds of Formula (I) with the proviso that R₃ is hydroxyl if R₁ is C₁-C₄ alkyl or methylthiomethyl and the compounds of Formula (II) are novel compounds.

The preferred compounds of Formula (I) are those in which R₁, R₂, R₃, R₄, R₅, R₆ and R₇ are as follows:
- R₁: is selected from methyl, ethyl, propyl, butyl, allyl, cyclopropyl, cyclobutyl, cyclopentyl, cyclohexyl, phenyl, benzyl, isopropyl, isobutyl, isoamyl, isovaleryl, 2-propynyl, 2-methoxymethyl, methylthiomethyl, hydroxyethyl, and methoxyethoxymethyl;
- R₂: is selected from methyl, ethyl, propyl, butyl, amyl, acetyloxy, propanoyloxy butanoyloxy, valeroyloxy, 2-propynyl and hydroxyl;
- R₃: is selected from methyl and hydroxyl;
- R₄: is selected from hydrogen, fluoro, and chloro;
- R₅: is selected from hydrogen, methyl, fluoro, and chloro;
- R₆: is selected from hydrogen, methyl, ethyl, and allyl; and
- R₇: is selected from hydroxyl, acetyloxy, and propanoyloxy.

The preferred compounds of Formula (II) are those in which R'₁, R'₂, R'₃, and R'₄, are as follows:
- R'₁: is selected from methyl, ethyl, and allyl;
- R'₂: is selected from hydroxyl, acetyloxy, and propanoyloxy;
- R'₃: is selected from methyl and hydroxyl; and
- R'₄: is selected from methyl, ethyl, and allyl.

The preferred subclasses of compounds within these preferred classes of compounds are as follows:
- Subclass 1:: compounds of Formula (I) wherein R₂, R₃, and R₄ are, respectively, hydroxyl, methyl, and fluoro; especially preferred are compounds of Examples 1-6 and 8-13 shown below;
- Subclass 2:: compounds of Formula (I) wherein R₅ is methyl; compounds of Subclass 1 are especially preferred;
- Subclass 3:: compounds of Formula (I) wherein R₃, R₄, and R₅ are, respectively, methyl, fluoro, and hydrogen; especially preferred are compounds of Examples 1-6 and 8-14 shown below;
- Subclass 4:: compounds of Formula (I) wherein R₂ and R₃ are hydroxyl and R₄ and R₅ are fluoro;
- Subclass 5:: compounds of Formula (I) wherein R₂ is selected from hydrogen and acyloxy, such as, acetyloxy, propanoyloxy, butanoyloxy, valeroyloxy, and 2-propynyl; compounds in which R₃ is hydroxyl and R₄ and R₅ are fluoro are especially preferred; and
- Subclass 6:: compounds of Formula (II) wherein R'₁ is selected from methyl, ethyl, and allyl; R'₂ is selected from hydroxyl, acetyloxy, and propanoyloxy; and R'₃ is methyl.

The following species are particularly preferred. The compound identified as number 7 is an especially preferred species of Subclass 6. The compounds are numbered in correspondence with the examples which follow the structures.

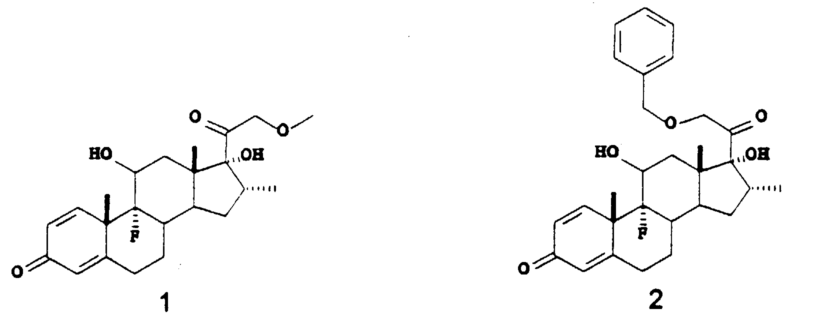

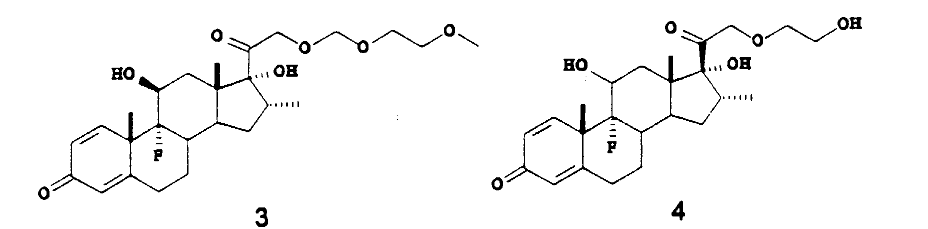

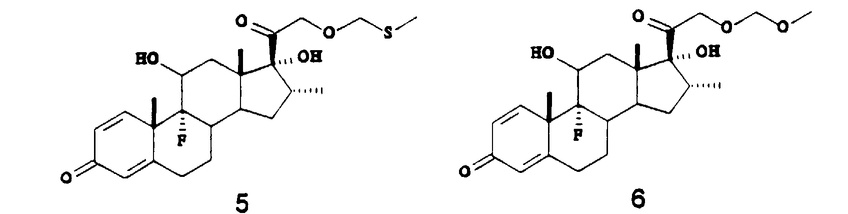

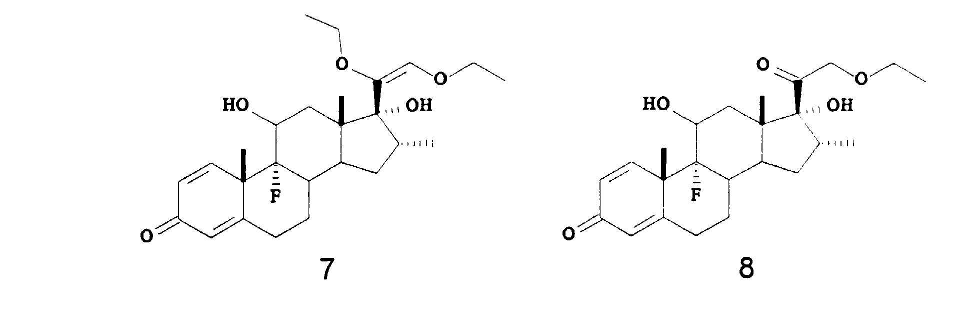

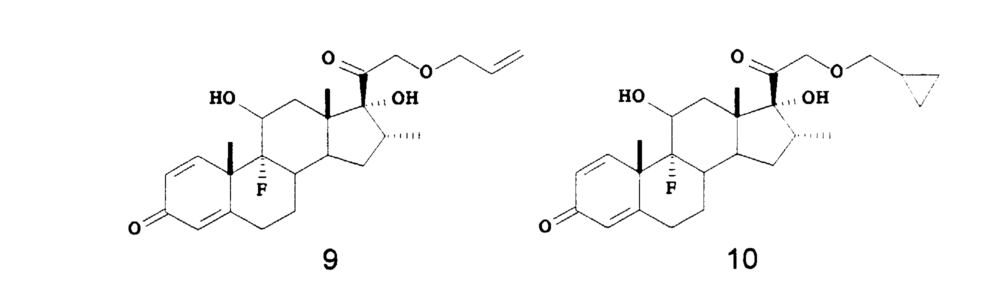

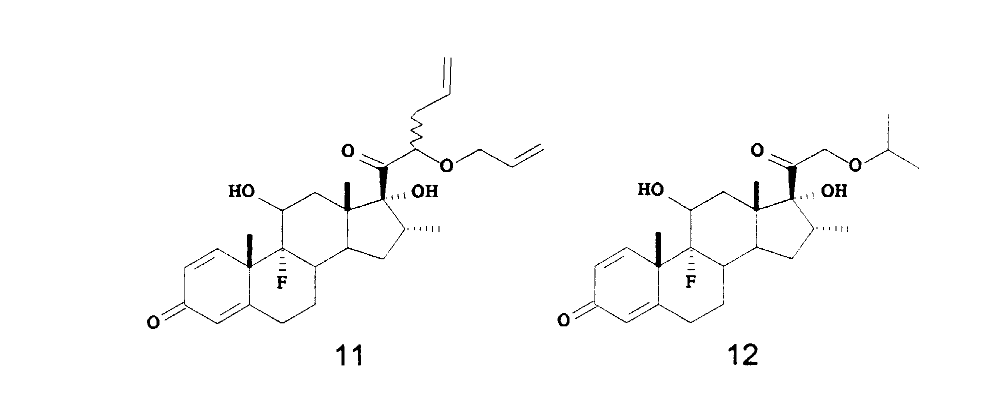

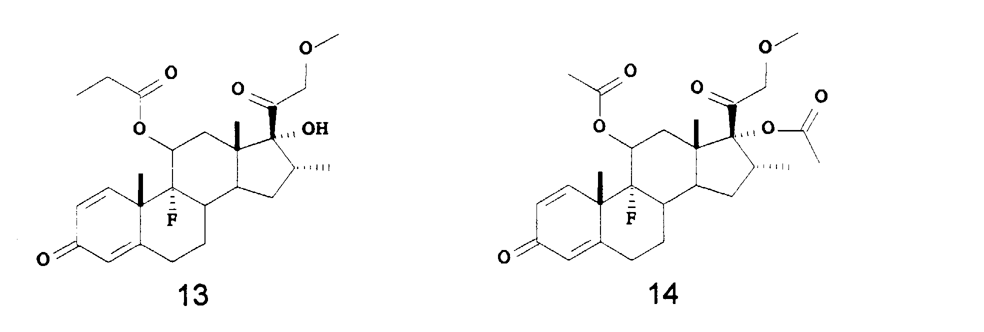

The above-described compounds are prepared from known starting materials using the Williamson ether synthesis or modifications thereof, such as: phase transfer catalysis; use of solid bases such as KOH, NaOH, or K₂CO₃, metal hydrides such as NaH, or organic bases such as N,N-diisopropylethylamine, tetramethylguanidine, or 1,8-diazabicyclo[5.4.0]-undec-7-ene in the presence of non-activated or activated halogenated alkylating reagents respectively under anhydrous conditions using aprotic solvents having high dielectric constants. This is further extended to the use of the steroid substrates as the electrophile, rather than as the nucleophile, such as is the case with tosylates, mesylates, or halides in syntheses of "S" or "O" ethers, or the preparation of ethers from metal catalyzed azide carbene/alcohols or, similarly, using the same reagents via fluoroboric or boron trifluoride etherate catalysis. Mono-ethers of the C21 methylene hydroxy group are preferred and are secondary to "C" and "O" bialkylation at C21 and/or "O" bialkylation at C20, C21.

The above-described dexamethasone ether derivatives of the present invention may be synthesized by means of liquid-liquid or liquid-solid phase transfer catalysis. As the name implies, phase transfer catalysis is carried out in a two-phase system; one phase is aqueous and the other is organic. In the alternative, a single anhydrous organic phase may contain a solid base, such as KOH, and either a quaternary amine or polyamine catalyst, such as tetramethylenediamine, in which the catalytic activity is at the surface of the base crystal. The catalyst utilized in the present invention is an alkylated quaternary amine, such as tetrabutylammonium sulfate, C₈-C₁₀ methyltrialkylammonium chloride, cetyltriethylammonium bromide, benzyl- triphenylphosphonium chloride, hexadecyltrimethylammonium bromide, tetrapentylammonium bromide, and tetrahexylammonium chloride. The reactants are: C₁-C₅ alkyl halides; branched chain alkyl halides such as isopropyl bromide; aryl halides such as benzyl bromide; and acetylenic halides such as propargyl bromide. The general mechanism of synthesis by means of phase transfer catalysis is as follows: first, the steroid anion complexes with the quaternary cation to form a lipophilic complex. The lipophilic complex is then transferred to the organic phase where it reacts with an alkyl halide to form an ether derivative of dexamethasone. The catalyst is then regenerated and passed back into the aqueous phase to react with the anion. The above-described method of synthesis is further illustrated by the examples set out below.

The above-described methods of synthesis are further illustrated by the examples set out below.

### Example 1: 9α-fluoro-11β,17α-dihydroxy-21-methoxy-16α-methylpregna-1,4-diene-3,20-dione

To a 50 milliliter (ml) 3-necked round bottom flask fitted with a magnetic stirrer, heating mantle, addition funnel, and thermometer was added 15 ml of toluene, 3.25 grams (g) of Adogen 464 (methyltrialkyl (C₈-C₁₀) ammonium chloride) (6.7 X 10⁻³ moles (mol)) and 0.5 g of sodium chloride (9 X 10⁻³ mol). The stirred mixture was warmed to 35°C, at which time 2 g of dexamethasone (5.1 X 10⁻³ mol) was slowly added, followed by 5 ml of toluene. The mixture was stirred for 5 minutes, followed by the addition of 3 ml of 50% sodium hydroxide solution (2.5 X 10⁻² mol). This mixture was equilibrated for 1-2 minutes, then 1.65 ml of iodomethane (2.62 X 10⁻² mol, 5.1 equivalents) was added dropwise, which elevated the temperature to 37°C. Within 3 to 5 minutes following the addition of iodomethane, the yellow ether precipitated from the solution. The reaction was allowed to continue for an additional 20 minutes. At that time, the flask contents were poured into 200 ml of 5% sodium chloride solution in a separatory funnel, gently shaken, and the layers allowed to separate. The lower, highly alkaline, phase was drawn off and the organic phase was washed with well-mixed 20% volume/volume (v/v) toluene/water (4 X 200 ml). The washed organic phase, left in the separatory funnel for 1 hour, underwent a secondary phase separation into clear yellow-brown upper layer (Adogen 464 catalyst) and a cloudy yellow lower layer. The lower layer was run into 250 ml of ice-cold ethyl ether which precipitated the white product. Precipitation of the product was completed by overnight refrigeration at 4°C. Residual Adogen catalyst was removed from the precipitated, filtered product with ether/toluene (200 ml) followed by ethyl ether (200 ml) and dried at 60°C. The product was further purified by silica-gel chromatography using ethyl acetate:hexane (1:1) as the eluant. M.P.=240-241°C (decomposition), [α]_{D} = 68.3° (1% dimethylformamide).

Calculated for C₂₃H₃₁FO₅: C=67.96%, H=7.70%. Found: C=67.8%, H=7.71%; Cl/MS, M/z=406.

¹H NMR(DMSO-d₆): δ 0.76 (d, J=7, 3H) (C16 αCH₃); 0.85 (s, 3H) (C18-CH₃); 1.45 (s, 3H) (C₁₉-CH₃); 3.24 (s, 3H) (OCH₃); 4.15 (br. s, 1H) (C11 αH); 4.26(AB, J=18.4, Δν=84, 2H) (C21-CH₂); 4.99 (s, 1H) (C17 αOH); 5.25 (br. s, 1H) (C11 βOH); 5.99 (s, 1H) (H-4); 6.20 (dd, J=10.1, J=1.9, 1H) (H-2); 7.27 (d, J=10.2, 1H) (H-1). (All coupling constants for all examples are reported in Hz.)

### Example 2: 9αfluoro-11β,17αdihydroxy-21-benzyloxy-16α-methylpregna-1,4-diene-3,20-dione

In a manner similar to that of Example 1, 1.298 g of Adogen 464 (2.67 X 10⁻³ mol), 5 ml of toluene, 0.6 ml of a 50% sodium hydroxide solution (5.2 X 10⁻³ mol), and 9.5 ml of water was added to a 50 ml 3-necked round bottom flask. The rapidly stirred mixture was warmed to 24°C, at which time 2.0 g of dexamethasone (5.1 X 10⁻⁹ mol) was added slowly and the mixture allowed to equilibrate for 10 minutes. Benzyl bromide, 4.362 g (2.55 X 10⁻² mol), was added dropwise over a period of 73 seconds. After 2 hours, the mixture was poured into 50 ml of ice-cold ethyl ether and the yellow precipitated product stirred for 15 minutes to remove the catalyst from the precipitate. The filtered precipitate was washed with ice-cold ether (4 X 100 ml) followed by with ice-cold absolute ethanol (4 X 50 ml) to afford pure white crystals of the product. The product was dried overnight at 60°C. Crystallization was from absolute ethanol. M.P.=243-245°C (decomposition). Calculated for C₂₉H₃₅FO₅: C=72.19%, H=7.33%. Found: C=72.19%, H=7.34%; Cl/MS, M/z=482.

¹H NMR(DMSO-d₆): δ 0.789 (d, J=7.1, 3H) (C16 αCH₃); 0.898 (s, 3H) (C18-CH₃); 1.48 (s, 3H) (C19 CH₃); 4.29 (br. s, 1H) (C11 αH); 4.59 (AB, J=18.5, Δν=75.2, 2H) (C21-CH₂-O-); 4.68 (d, J=3.0, 2H) (C21 -O-CH₂-); 5.02 (s, 1H) (C17 αOH); 5.27 (br. s, 1H) (C11 βOH); 6.00 (s, 1H) (H-4); 6.22 (dd, J=10.1, J=1.8, 1H) (H-2); 7.29 (d, J=10, 1H) (H-1); 7.35 (m, 5H)(C21 O-C-Ph).

### Example 3: 9α-fluoro-11β,17α-dihydroxy-21-(2-methoxyethoxy)methoxy-16α-methylpregna-1,4-diene-3,20-dione

Dexamethasone, 1.6 g (4.1 X 10⁻³ mol), was combined with 35 ml of methylene chloride, 1.07 ml (1.5 equivalents) of N,N'-diisopropylethylamine (NDEA) and 0.7 ml (6.13 X 10⁻³ mol, 1.5 equivalents) of 2-methoxyethoxymethylchloride (MEMCL) in a 50 ml flask under rapid magnetic stirring at ambient temperature. This mixture remained as a suspension. After 1.5 hours, an additional equivalent each of MEMCL and NDEA were added and the reaction allowed to proceed for an additional 3.75 hours, after which time a solution was obtained. The flask contents were then washed with 100 ml of 5% HCI in a separatory funnel. The lower organic phase was then removed and washed with water (4 X 100 ml). The organic phase was dried with MgSO₄, then combined, heated to the boiling point with 3 g of a celite:activated carbon mixture (1:1), cooled, and filtered. The clarified organic phase was evaporated to dryness and recrystallized from ethyl acetate. M.P.=193-195°C (decomposition).

Calculated for C₂₆H₃₇FO₇: C=64.98% H=7.78%. Found: C=65.25%, H=8.06%; Cl/MS, M/z=480.

¹H NMR (DMSO-d₆): δ 0.718 (d, J=7.1, 3H) (C16 αCH₃); 0.866 (s, 3H) (C18-CH₃); 1.46 (s, 3H) (C₁₉-CH₃); 3.25 (s, 3H) (OCH₃); 3.52 (dt, J=4.8, 4H) (C21-O-(CH₂)₂-O); 4.42 (AB, J=18.4, Δν=74.5, 2H) (C21 -CH₂-O-); 4.66 (s, 2H) (C21 -O-CH₂-O); 5.04 (s, 1H) (C17 αOH); 5.33 (br. d, J=4, 1H) (C11 βOH); 6.00 (s, 1H) (H-4); 6.22 (dd, J=10.1, J=1.8, 1H) (H-2); 7.29 (d, J=10, 1H) (H-1).

### Example 4: 9 α-fluoro-11β,17α-dihydroxy-21-(2-hydroxy)ethoxy-16α-methylpregna-1,4-diene-3.20-dione

To a 50 ml 3-necked round bottom flask, fitted with a magnetic stirrer and a nitrogen source was added 10 ml of dry dimethylformamide (DMF), 2.83 g (1.88 X 10⁻² mol, 1.25 equivalents) of t-butyldimethylsilylchloride (TBDMS) and 3 g of imidazole (4.4 X 10⁻² mol) and stirred until all reagents were solubilized. 2-Bromoethanol, 1.87 g (1.5 X 10⁻² mol), was added dropwise and the reaction allowed to proceed for 4.25 hours at ambient temperature. The reaction mixture was combined with 400 ml of ether and 150 ml of water and then washed with water (4 X 200 ml). The organic phase was dried with MgSO₄ and evaporated down to a clear, off-white oil. Residual solvent was removed under high vacuum overnight. The reaction product, 2-bromoethanol silyl ether 1 was used without further purification in the subsequent alkylation reaction. In a 50 ml 3-necked round bottom flask fitted with a magnetic stirrer, heating mantle, and a nitrogen source, 0.5 g of dexamethasone (1.28 X 10⁻³ mol), and 0.8 g of 1 (3.35 X 10⁻³ mol, 2.62 equivalents) were dissolved in 3 ml of hexamethyl-phosphoramide at 30°C. The reaction was started by the addition of 0.22 g (80%) of solid potassium hydroxide (3.14 X 10⁻³ mol) and allowed to proceed for 70 minutes. An additional 1.64 equivalents of 1 was then added and the reaction allowed to proceed for an additional 60 minutes with the heat off. Under these conditions, the mono- and the bi-alkylated products formed with little or no degradation of the starting substrate. The reaction did not go to completion. The mono-alkylated steroid product 2 was partitioned between ethyl acetate/water (50mV25ml) and the organic phase washed with water (4 X 50 ml). The product 2 was purified by silica gel chromatography using ethyl acetate:hexane (55:45) as the eluant. Cl/MS gave the anticipated M/z = 550 molecular ion and the correct H-NMR assignments for C21 "O" alkylation.

Silyl deprotection of 2 was carried out in a 50 ml 3-necked round bottom flask containing 0.080 g of 2 (1.45 X 10⁻⁴ mol) and 0.155 g of tetrabutylammonium fluoride (5 X 10⁻⁵ mol, 3 equivalents) in 2 ml of dry tetrahydrofuran for 20 minutes at ambient temperature. The deprotected, alkylated steroid 3 was partitioned into ethyl acetate/water (25ml/15ml) and the organic phase washed with water (4 X 15 ml), dried with MgSO₄, and evaporated to dryness. Crystallization was from acetone:hexane. M.P.= 228-230°.

Calculated for C₂₄H₃₃FO₆: C=66.05%, H=7.56%. Found: C=65.92%, H= 7.94%; CI/MS, M/z=436.

¹H NMR (DMSO-d₆): δ 0.768 (d, J=7.1, 3H) (C16 αCH₃); 0.863 (s, 3H) (C18-CH₃); 1.47 (s, 3H) (C19-CH₃); 3.43 (m, 4H) (C21 -O-(CH₂)₂-O); 4.14 (br. s, 1H) (C11 αH); 4.36 (AB, J=18.6, Δν=82.7, 2H) (C21 -CH₂O-); 4.6 (dd, J=4.7, 1H) (C21 -O-C-C-OH); 4.99 (s, 1H) (C17 αOH); 5.27 (br. s, 1H) (C11 βOH); 5.99 (s, 1H) (H-4); 6.21 (dd, J=10, J=2, 1H) (H-2); 7.28 (d, J=10.3, 1H) (H-1).

### Example 5: 9α-fluoro-11β,17α-methyl-dihydroxy-21-(methylthio)methoxy)-16α-methylpregna-1,4-diene-3,20-dione

Dexamethasone, 2 g (5.1 X 10⁻³ mol), was added to a solution of 16.4 ml of dimethylsulfoxide (0.230 mol), 10.6 ml of acetic anhydride (0.1122 mol), and 3.2 ml of acetic acid (0.0561 mol) in a 100 ml 3-necked round bottom flask equipped with a magnetic stirrer and allowed to react for 24 hours at ambient temperature. The mixture was then neutralized by very slowly pouring the contents of the flask into 150 ml of 10% sodium carbonate solution and then adding 300 ml of ethyl acetate. The organic phase was washed successively with ethyl acetate saturated water (4 X 300 ml) and brine, and dried with MgSO₄. The evaporated organic base was purified by silica-gel chromatography using ethyl acetate:hexane (1:1) as the eluant. The product was crystallized from 30% ethanol. M.P.=221-222°C (decomposition).

Calculated for C₂₄H₃₃FO₅S: C=63.72%, H=7.30%. Found: C=63.71%, H = 7.36%; CI/MS, M/z=452.

¹H NMR (DMSO-d₆): δ 0.784 (d, J=7.1, 3H) (C16 αCH₃); 0.865 (s, 3H) (C18-CH₃); 1.42 (s, 3H) (C₁₉-CH₃); 2.09 (s, 3H) (C21 -S-CH₃); 4.16 (br. d, 1H) (C11 αH); 4.46 (AB, J=16, Δν=76.4, 2H) (C21 CH₂); 4.67 (s, 2H) (O-CH₂S); 5.07 (s, 1H) (C17 OH); 6.0 (s, 1H) (H-4); 6.24 (dd, J=10.1, J=1.7, 1H) (H-2); 7.22 (d, J=10.2, 1H) (H-1).

### Example 6: 9α-fluoro-11β,17α-dihydroxy-21-(methoxy)methoxy-16α-methylpregna-1,4-diene-3,20-dione

Dexamethasone, 2 g (5.1 X 10⁻³ mol), was dissolved in a solution of 10 ml of dry dimethylsulfoxide and 1.8 ml (2 equivalents) of N,N-diisopropylethylamine in a 50 ml 3-necked round bottom flask equipped with a magnetic stirrer under nitrogen. Chloromethoxymethyl ether, 0.775 ml (1.02 X 10⁻² mol, 2 equivalents), in a 1 ml Hamilton syringe, was added below the surface of the solution over a period of 1 minute, during which time the temperature rose from 23° to 29°C. The reaction proceeded over 105 minutes, after which time an additional equivalent of chloromethoxymethyl ether was added. The reaction was continued for 18 hours, then partitioned into ethyl acetate/water 400mV200 ml), and the organic phase washed with water (2 X 200 ml), 200 ml of 1% sodium hydroxide solution, water (4 X 200 ml), and brine. The organic phase was dried with MgSO₄ and evaporated to dryness. The product was separated and purified by silica gel chromatography using ethyl acetate:hexane (65:35) as the eluant. Crystallization was from acetone:hexane. M.P.=243-244°C (decomposition).

Calculated for C₂₄H₃₃FO₆: C=66.06%, H=7.50%. Found: C=65.71%, H = 7.57%; CI/MS, M/z=436.

¹H NMR (DMSO-d₆): δ 0.825 (d, J=13.2, 3H) (C16 αCH₃); 0.866 (s, 3H) (C18-CH₃); 1.46 (s, 3H) (C19-CH₃); 3.27 (s, 3H) (C21 -O-CH₃); 4.13 (br. s, 1H) (C11 αH); 4.41 (AB, J=18.4, Δν=73.5, 2H) (C21 CH₂); 4.57 (s, 2H) (C21 -O-CH₂-O); 5.04 (s, 1H) (C17 αOH); 5.34 (s, 1H) (C11 βOH); 6.0 (s, 1H) (H-4); 6.25 (dd, J=10.1, J=2, 1H) (H-2); 7.32 (d, J=10.0, 1H) (H-1).

### Example 7: 9α-fluoro-11β,17αdihydroxy-Δ²⁰-ethoxy-21-ethoxy-16α-methylpregna-1,4-diene-3,20-dione

Potassium hydroxide was powdered in a hot mortar (75°C) and 0.912 g (87.8%, 1.6 X 10⁻² mol) was added to 10 ml of dry dimethyl sulfoxide in a 50 ml 3-necked round bottom flask equipped with a magnetic stirrer and attached to a nitrogen source. After a 5 minute equilibration, 2 g of dexamethasone (5.1 X 10⁻³ mol) was added and, after dissolution, 2.4 g of iodomethane (1.52 X 10⁻² mol, 3 equivalents) was added dropwise and the reaction allowed to proceed for 20 minutes at ambient temperature. The reaction was partitioned into ethyl acetate/water (100ml/50ml) and the organic phase washed with ethyl acetate saturated water (2 X 50 ml) and brine. The organic phase was dried with MgSO₄, evaporated to dryness, separated, and partially purified by silica gel chromatography using ethyl acetate:hexane (1:1) as the eluant. The analytical sample was crystallized repeatedly from acetone:hexane. M.P.=176-177°C (decomposition).

Calculated for C₂₆H₃₇FO₅: C=69.64%, H=8.25%. Found: C=69.73%, H=8.31%; Cl/MS, M/z=448.

¹H NMR (DMSO-d₆): δ 0.865 (d, J=7.1, 3H) (C16 αCH₃); 0.942 (s, 3H) (C18-CH₃); 1.49 (s, 3H) (C19-CH₃); 1.56 (t, 3H) (C21 -O-C-CH₃); 1.76 (t, 3H) (C20-O-C-CH₃); 3.69 (s, 1H) (C17αOH); 3.71 (q, 2H) (C21 O-CH₂-C); 3.92 (q, 2H) (C20-O-CH₂-C); 4.09 (br.d, J=14, 1H) (11αH); 5.16 (s, 1H) (C11 βOH); 5.75 (s, 1H)(C21 enol H); 6.00 (s, 1H) (H-4); 6.21 (dd, J=10, J=1.7, 1H) (H-2); 7.22 (s, J=10.2, 1H) (H-1).

### Example 8: 9α-fluoro-11β,17α-dihydroxy-21-ethoxy-16α-methylpregna-1,4-diene-3,20-dione

Sodium hydride (60% dispersion), 0.061 g (2.55 X 10⁻³ mol), was added to 10 ml of benzene in a 250 ml 3-necked round bottom flask fitted with a magnetic stirrer, nitrogen source, reflux condenser, and a dropping funnel. One g of dexamethasone (2.55 X 10⁻³ mol) dissolved in 50 ml of dry DMF was added dropwise over a period of 20 minutes, after which time the color changed from a light yellow to a dark yellow. Evolution of hydrogen gas, chased continuously by nitrogen, ceased at this point. At the cessation of hydrogen gas evolution, 3 equivalents of iodomethane (7.7 X 10⁻³ mol) was added to the rapidly stirred solution and the reaction allowed to proceed for 30 minutes at ambient temperature. The reaction products were partitioned into ethyl acetate:water (500ml/500 ml) and the organic layer washed with water (3 X 100 ml) and brine. The organic phase was dried with sodium sulfate and evaporated down to a clear yellow oil. The oil was dissolved in ethyl acetate:hexane (1:1) and the product separated by silica gel chromatography. The product was crystallized from acetone:hexane. M.P.=239-241°C (decomposition).

Calculated for C₂₄H₃₃FO₅: C=68.55%, H=7.93%. Found: C=68.59%, H=7.99%; Cl/MS, M/z=420.

¹H NMR (DMSO-d₆): δ 0.775 (d, J=8.4, 3H) (C16 αCH₃); 0.864 (s, 3H) (C18-CH₃); 1.48 (s, 3H) (C19-CH₃); 1.12 (t, J=6.9, 2H) (C21 -O-C-CH₃); 3.39 (q, J=5.6, 2H) (C21 -O-CH₂-C); 4.11(br. s, 1H) (C11 αH); 4.32 (AB, J=18.5, Δν=81.5, 2H); (C21 CH₂); 5.00 (s, 1H) (17 αOH); 5.28 (s, 1H) (11 βOH); 6.00 (s, 1H) (H-4); 6.22 (dd, J=10.2, J=1.8, 1H) (H-2); 7.29 (d, J=10.1, 1H) (H-1).

### Example 9: 9α-fluoro-11β,17α-dihydroxy-21-allyloxy-16α methylpregna-1,4-diene-3,20-dione

Powdered potassium hydroxide (87.7%), 0.456 g (8.14 X 10⁻³ mol), was added to 10 ml of dry dimethylsulfoxide in a 50 ml 3-necked round bottom flask fitted with a magnetic stirrer and a nitrogen source. After the contents were stirred for 5 minutes, 1 g of dexamethasone (2.55 X 10⁻³ mol) was added slowly until dissolved. Allybromide, 0.32 g (2.64 X 10⁻³ mol, 1 equivalent) was added dropwise over 1 minute and the reaction allowed to proceed for 10 minutes. The reaction products (minus solid potassium hydroxide) were transferred with ethyl acetate and partitioned into ethyl acetate/water (100ml/200 ml). The organic layer was washed with water (3 X 100 ml), brine (2 X 15 ml), dried with MgSO₄, and evaporated to dryness. The product was separated and purified by silica gel chromatography using ethyl acetate:hexane (1:1) as the eluant. The product was crystallized from acetone: hexane. M.P.=245.5-248.5°C (decomposition).

Calculated for C₂₅H₃₃FO₅: C=69.44%, H=7.64%. Found: C=69.59%, H=7.87%; CI/MS, M/z=432.

¹H NMR (DMSO-d₆): δ 0.779 (d, J=7.1, 3H) (C16 αCH₃); 0.872 (s, 3H) (C18-CH₃); 1.49 (s, 3H) (C19-CH₃); 3.95 (q, 2H) (C21 -C-O-CH₂); 4.19 (br. s, 1H) (C11 αH); 4.55 (AB, J=18.5, Δν=85.2, 2H) (C21 CH₂); 5.00 (s, 1H) (C17 αOH); 5.23 (m, 2H) (C21-C=CH₂); 5.89 (m, 1H) (C21 -CH=C); 6.00 (s, 1H) (H-4); 6.22 (dd, J=10.1, J=1.8, 1H) (H-2); 7.27 (d, J=10.1, 1H) (H-1).

### Example 10: 9α-fluoro-11β,17α-dihydroxy-21-cyclopropylmethoxy-16α-methylpregna-1,4-diene-3,20-dione

Powdered potassium hydroxide (87.7%), 0.456 g (8.14 X 10⁻³ mol), was added to 10 ml of dry dimethylsulfoxide in a 50 ml 3-necked round bottom flask fitted with a magnetic stirrer and a nitrogen source. After the flask contents were stirred for 10 minutes, 1 g of dexamethasone (2.55 X 10⁻³ mol) was added slowly until dissolved. Cyclopropylmethylbromide, 0.342 g (2.58 X 10⁻¹⁰ mol, 1 equivalent), was added dropwise over 1 minute and the reaction allowed to proceed for 15 minutes at ambient temperature. The reaction products (minus solid potassium hydroxide) were transferred with ethyl acetate and partitioned into ethyl acetate/water (100mV200 ml). The organic phase was washed with water (3 X 200 ml), brine (2 X 15 ml), dried with MgSO₄ and evaporated to dryness. The product was separated and purified by silica gel chromatography using ethyl acetate:hexane (7:3) as the eluant. M.P.=238.5-239.5°C (decomposition).

Calculated for C₂₆H₃₅FO₅: C=69.96%, H=7.85%. Found: C=69.78%, H=7.99%; Cl/MS, M/z=446.

¹H NMR (DMSO-d₆): δ 0.323 (m, 5H) (C21 -C-CH-cyc(C₃H₅)); 0.775 (s, 3H) (C16αCH₃); 0.863 (s, 1H) (C18-CH₃); 1.49 (s, 3H) (C19-CH₃); 3.23 (dd, J=2.3, J=6.8, 2H) (C21 -O-CH₂-cyc(C₃H₅)); 4.11(br. d, J=8.3, 1H) (C11 αH); 4.34 (AB, J=18.5, Δν=82.2, 2H) (C21 CH₂-O-); 4.98 (s, 1H) (C17 αOH); 5.27 (s, 1H) (C11 βOH); 6.00 (s, 1H) (H-4); 6.22 (dd, J=10.1, J=1.87, 1H) (H-2); 7.29 (d, J=10.2, 1H) (H-1).

### Example 11: 9α-fluoro-11β,17α-dihydroxy-21-allyl-21-allyloxy-16α-methyl-1,4-diene-3,20-dione

In a manner similar to that of Example 1, 14 ml of toluene and 1.68 g of Adogen 464 (3.42 X 10⁻³ mol) was added to a 50 ml 3-necked round bottom flask and warmed to 32°C. At this temperature, 1.5 g of 50% sodium hydroxide solution (3.75 X 10⁻² mol) was added and the flask contents equilibrated for 5 minutes. Dexamethasone, 1 g (2.55 X 10⁻³ mol), was added slowly until dissolved and equilibrated for 3 minutes. Allylbromide, 0.924 g (7.6 X 10⁻³ mol, 3 equivalents), was added dropwise over 1 minute and the reaction allowed to proceed for 20 minutes at 32°C. The reaction contents were partitioned into 300 ml of ethyl acetate/water 300ml/400 ml). The organic phase was washed with water (2 X 400 ml), brine (2 X 15 ml), dried with MgSO₄, and evaporated down to a clear, slightly yellow, glass. The product was isolated by silica gel chromatography using ethyl acetate:hexane as the eluant. No M.P. - product amorphous.

Calculated for C₂₈H₃₇FO₅: C=71.19%, H=7.85%. Found: C=70.45%, H=7.83%; Cl/MS, M/z=472.

¹H NMR (DMSO-d₆): δ 0.871 (d, J=7.1, 3H) (C16 αCH₃); 0.949 (s, 3H) (C18-CH₃); 1.49 (s, 3H) (C19-CH₃); 4.15 (br. d, 1H) (C11 αH); 4.2 (br. m, 5H) (C21 -allyl); 4.88 (s, 1H) (C17 OH); 5.14 (br. m, 5H) (C21 -allyl); 5.33 (br. s, 1H) (C11 βOH); 5.81 (m, 2H) (C21 -allyl); 6.01 (s, 1H) (H-4); 6.22 (dd, J=9.9, J=1.8, 1H) (H-2): 7.28 (d, J=10.1, 1H) (H-1).

### Example 12: 9α-fluoro-11β,17α-hydroxy-21-isopropyloxy-16α-methylpregna-1,4-diene-3,20-dione

Powdered potassium hydroxide (87.7%), 0.456 g (8.14 X 10⁻³ mol), was added to 7 ml of dry dimethylsulfoxide in a 50 ml 3-necked round bottom flask equipped with a magnetic stirrer and a nitrogen source. The reaction was allowed to equilibrate for 5 minutes, then 1 g of dexamethasone (2.55 X 10⁻³ mol) was added slowly until dissolved. 2-Bromopropane, 0.914 g (7.65 X 10⁻³ mol, 3 equivalents), was added dropwise over 1 minute and the reaction allowed to proceed for 15 minutes at ambient temperature. The reaction solution was partitioned into ethyl acetate/water (105 ml/75 ml) and the organic phase washed with ethyl acetate saturated water (3 X 50 ml) and brine (2 X 15 ml). The organic phase was dried with MgSO₄, evaporated to dryness, and partially purified by silica gel chromatography using acetone:hexane (7:3) as the eluant. The product was crystallized repeatedly from acetone:ether. M.P. = 232-233.5°C (decomposition).

Calculated for C₂₅H₃₅FO₅: C=69.10%, H=8.12%. Found: C=69.02%, H=8.32%; Cl/MS molecular ion, M/z=434.

¹H NMR (DMSO-d₆): δ 0.785 (d, J=7.2, 3H) (C16 αCH₃); 0.861 (s, 3H) (C18 CH₃); 1.07 (m, 6H) (C21 -O-C-(CH₃)₂); 1.48 (s, 3H) (C19 CH₃); 3.55 (m, 1H) (C21 -O-CH-(CH₃)₂); 4.11(br. s, 1H) (C11-H); 4.32 (AB, J=18.5, Δν=81.5, 2H) (C21 CH₂-O-); 4.98 (s, 1H) (C17 αOH); 5.29 (br. t, 1H) (C11 βOH); 6.03 (s, 1H) (H-4); 6.22 (dd, J=10.1, J=1.8, 1H) (H-2); 7.28 (d, J=10.2, 1H) (H-1).

### Example 13: 9α-fluoro-11β-propionoxy-17α-hydroxy-21-methoxy-16α-methylpregna-3,20-dione

The compound of Example 1, 0.8 g(1.97 X 10⁻³ mol), was suspended in 5 ml of N,N-dimethylformamide and 25 ml of pyridine in a 250 ml 3-necked round bottom flask equipped with a magnetic stirrer and nitrogen source. Propionic anhydride, 0.76 ml (3 equivalents), and 0.040 g (0.016 equivalent) of 4-dimethylaminopyridine (DMAP) were added and the mixture stirred for 2 hours at ambient temperature. The substrate at this point remained in suspension. After 3 hours, an additional equivalent of DMAP (0.24 g) and 7 equivalents (1.8 ml) of propionic anhydride were added and the reaction allowed to proceed for 16 hours at ambient temperature. The clear, light yellow solution was equilibrated with 200 ml ethyl acetate and 100 ml of 4% HCI. The organic phase was washed sequentially with 80 ml of 5% HCI, 3 X 80 ml of 3% HCI, solution and 200 ml of water. The light brown organic phase was dried with brine and MgSO₄, followed by charcoal-celite in hot ethyl acetate, and evaporated down to a clear glass. Trituration with ethyl ether, ethyl ether/hexane gave a white powder which was twice crystallized from ethyl acetate/isopropyl ether to give very fine needles. NMR indicated that the product crystallized as an ethyl acetate solvate of approximately 1/2 mol. M.P.=178-178.5; M/z = 462.

Calculated for C₂₆H₃₅FO₆•1/2(C₄H₈O₂): C=66.38%, H=7.75%. Found C= 66.74%, H=7.96%.

¹H NMR (DMSO-d₆): δ 0.79 (d, J=7.0, 3H) (C16 CH₃); 0.79 (s, 3H) (C18 CH₃); 1.4 (s, 3H) (C19 CH₃); 2.4 (q, J=7.5, 2H) (C11 βCOCH₂-C); 3.2 (s, 3H) (C21 -O-CH₃); 4.1 (t, J=7.5, 3H) (C11 βCO-C-CH₃); 4.26 (AB, J=18.4, Δν=81.0) (C21 CH₂); 5.17 (s, 1H) (C17 αOH); 5.22 (m, 1H) (C11 αH); 6.1 (s, 1H) (H4); 6.3 (dd, J=1.8, J=10.1, 1H) (H2); 6.8 (d, J=10.1, 1H) (H1).

### Example 14: 9α-fluoro-11β-17α-diacetoxy-21-methoxy-16α-methylpregna-1,4-diene-3,20-dione

The compound of Example 1, 1.0 g (2.46 X 10⁻³ mol), was combined with 5 ml of glacial acetic acid and 5 ml of acetic anhydride under nitrogen in a 50 ml, 3-necked round bottom flask equipped with a magnetic stirrer in an 49°C oil bath. When the temperature reached 79 - 81°C, 0.233 g of p-toluenesulfonic acid monohydrate (0.5 equivalent) was added and the reaction allowed to proceed for 5.5 hours. The reaction contents were suspended in ethyl acetate/water (200 mV200 ml) and the organic phase washed with water (6 X 200 ml) to neutrality. The organic phase was dried with MgSO₄ and by-products removed with charcoal-celite in hot ethyl acetate. The product was isolated on 200 g of silica gel using ethyl acetate:toluene (4:7) as the eluant. M.P. 120°C - 123°C; M/z = 490.

Calculated for C₂₇H₃₅FO₇; C = 66.10%, H = 7.19%. Found C= 66.24%, H= 7.61%.

¹H NMR (DMSO-d₆): δ 0.85 (s, 3H) (C18 CH₃); 0.86 (d, J=6.7, 3H) (C16 αCH₃); 1.37 (s, 3H) (C19 CH₃); 2.05 (s, 3H) (COCH₃); 2.11 (s, 3H) (COCH₃); 3.26 (s, 3H) (C21 -O-CH₃); 4.1 (s, 2H) (COCH₂-O); 5.2 (m, 1H) (C11 αH); 6.1 (s, 1H) (H4); 6.3 (dd, J=1.8, J=10.1, 1H) (H2); 6.82 (d, J=10.1, 1H) (H1).

The steroid derivatives of Formula (I) and (II) may be incorporated into various types of ophthalmic pharmaceutical compositions for delivery to the eye. For example, one or more of these compounds may be combined with ophthalmologically acceptable preservatives, surfactants, viscosity enhancers, buffers, sodium chloride and water to form an aqueous, sterile ophthalmic suspension.

In order to prepare sterile ophthalmic ointment formulations, one or more of the compounds is combined with a preservative in a hydrophilic/hydrophobic base such as mineral oil, liquid lanolin, or white petrolatum. Sterile ophthalmic gel formulations may be prepared by suspending the active ingredient in a hydrophilic base prepared from the combination of Carbopol®-940 (a carboxy vinyl polymer available from the B. F. Goodrich Company), sodium hydroxide, sodium chloride, and preservatives. The specific type of formulation selected will depend on various factors, such as the type of inflammation being treated (e.g., internal or external) and dosage frequency. Ophthalmic suspensions, ointments, and gels are the preferred dosage forms.

In addition, the formulations will normally include conventional ingredients such as preservatives, tonicity agents, and so on. The steroid derivatives of Formula (I) and (II) will normally be contained in these formulations in an amount in the range of 0.05% to 2.0% by weight.

The formulations described in the following example further illustrate the dosage forms which may be utilized in the present invention.

### Example 15

In the following formulations, the term "steroid" represents any of the above-described C21 steroid derivatives of Formula (I) and (II), including the following specific example of such derivatives: 9α-fluoro-11β,17α-dihydroxy-21-methoxy-16α-methylpregna-1,4-diene-3,20-dione.

| Suspension | |
|---|---|
| Ingredient | Percentage by weight |
| Steroid | .05 - 2.0 |
| Benzalkonium Chloride | 0.001 - 0.02 |
| Polysorbate-80 or Tyloxopol | 0.01 - 1.0 |
| Phosphate Buffer pH | 5 - 100mMol |
| Sodium Chloride | 0 - 0.9 |
| Hydroxypropyl methyl cellulose | 0.1 - 0.5 |
| Water | q.s. |

| Ointment | |
|---|---|
| Ingredient | Percentage by weight |
| Steroid | 0.05 - 2.0 |
| Chlorobutanol | 0.5 |
| Methyl or propyl parabens | 0.01 - 0.1 |
| Mineral Oil | 0 - 10 |
| Liquid Lanolin | 0 - 10 |
| White petrolatum | q.s. |

| Gel | |
|---|---|
| Ingredient | Percentage by weight |
| Steroid | 0.05 - 2.0 |
| Carbopol®-940 | 1 - 4 |
| Sodium Hydroxide | (q.s.) pH: 4.5 - 8.0) |
| Sodium Chloride | 0 - 0.9 |
| Water | q.s. |

The treatment method of the present invention comprises applying an ophthalmic pharmaceutical composition containing an anti-inflammatory effective amount of one or more steroid derivatives of Formula (I) or (II) to the affected ocular tissue when indicated for the relief of inflammation.

Examples of ophthalmic inflammatory disorders which can be treated by the compositions of the present invention include, but are not limited to: acute or chronic conjunctivitis; acute or chronic anterior segmental uveitis in normal individuals, steroid responders, and individuals with frank primary open and/or closed angle glaucoma; inflammation and IOP elevation resulting from intraocular lens implantation and/or inflammation; and IOP elevation following trabecular filtering or laser surgeries. The dosage regimen utilized will depend on various factors, such as the severity of the inflammation and the duration of action of the particular formulation utilized. In general, the above-described formulations may be topically applied, for example, as drops to the upper globe, or as a 0.5-1.0 cm strip of ointment or gel to the lower conjunctival sac of the eye. Suspensions will generally be applied two to four times daily, while ointments or gels will generally be applied once or twice daily. The application of sustained release formulations (e.g., polymer based gels) once daily at bedtime will be preferred for some conditions.

The above-described formulations are useful in treating virtually any type of ocular inflammation. These formulations are especially useful in the treatment of ocular inflammation in patients who are predisposed to experiencing elevated intraocular pressure when treated with a conventional steroid compound; this class of patients, which is estimated as constituting approximately 5% of the general population, is referred to as "steroid responders." The formulations also have special value in treating ocular inflammation in patients suffering from open angle glaucoma, since it has been estimated that approximately 92% of these patients experience a large increase in intraocular pressure after chronic (e.g., 6 to 8 weeks) steroid therapy. The formulations also find special use in treating the inflamed ocular tissue of patients who are predisposed to open-angle glaucoma or ocular hypertension.

The present invention has been described above in connection with certain preferred embodiments. However, as obvious variations thereon will become apparent to those skilled in the art, the invention is not to be considered as limited thereto.

## Claims

1. A compound of the formula: 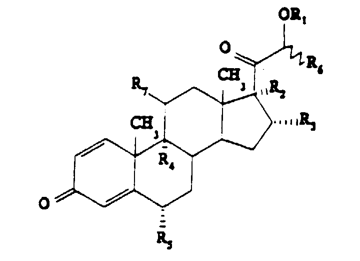 wherein:
R₁ is selected from substituted or unsubstituted: C₁-C₅ alkyl; C₃-C₆ (ring) and C₁-C₃ (alkyl) cycloalkyl and cycloalkylalkyl; aryl and arylalkyl, such as phenyl and benzyl; and C₂-C₅ alkenyl and alkynyl; wherein the substituent or substituents are selected from: C₁-C₆ alkoxy and alkyl; C₂-C₆ alkoxyalkyl, alkoxyalkoxyalkyl, alkylthioalkyl, hydroxyalkyl, aminoalkyl, and nitriloalkyl; C₄-C₈ (beta-carboxyalkoxy)alkyl; and C₂-C₆ alkenoxy and alkynoxy;
R₂ is selected from hydrogen and substituted or unsubstituted: C₁-C₆ alkyl, alkenyl and alkynyl; acyloxy and hydroxyl; wherein the substituent or substituents are selected from: C₁-C₃ alkyl; hydroxyl; and halogen (F, Cl and Br);
R₃ is selected from methyl and hydroxyl;
R₄ is selected from hydrogen, fluoro, and chloro;
R₅ is selected from hydrogen, methyl, fluoro, and chloro;
R₆ is selected from hydrogen, methyl, ethyl, and allyl; and
R₇ is selected from hydroxyl, acetyloxy, propanoyloxy, and butanoyloxy;
or a pharmaceutically acceptable salt thereof, with the proviso that R₃ is hydroxyl, if R₁ is C₁-C₄ alkyl or methylthiomethyl.

2. The compound of claim 1, wherein:
R₁ is selected from methyl, ethyl, propyl, butyl, allyl, cyclopropyl, cyclobutyl, cyclopentyl, cyclohexyl, phenyl, benzyl, isopropyl, isobutyl, isoamyl, isovaleryl, 2-propynyl, 2-methoxymethyl, methylthiomethyl, hydroxyethyl, and methoxyethoxymethyl;
R₂ is selected from hydrogen, methyl, ethyl, propyl, butyl, amyl, acetyloxy, propanoyloxy, butanoyloxy, valeroyloxy, 2-propynyl, and hydroxyl;
R₃ is selected from methyl and hydroxyl;
R₄ is selected from hydrogen, fluoro, and chloro;
R₅ is selected from hydrogen, methyl, fluoro, and chloro;
R₆ is selected from hydrogen, methyl, ethyl, and allyl; and
R₇ is hydroxyl.

3. The compound of claim 2, wherein R₂, R₃ and R₄ are, respectively, hydroxyl, methyl and fluoro.

4. The compound of claim 1, 2 or 3, wherein R₅ is methyl.

5. The compound of claim 3, wherein R₅ is hydrogen.

6. The compound of claim 2, wherein R₂ and R₃ are hydroxyl and R₄ and R₅ are fluoro.

7. The compound of claim 1, wherein R₂ is selected from hydrogen, acetyloxy, propanoyloxy, butanoyloxy, valeroyloxy and 2-propynyl.

8. The compound of claim 7, wherein R₃ is hydroxyl and R₄ and R₅ are fluoro.

9. A compound of the formula: 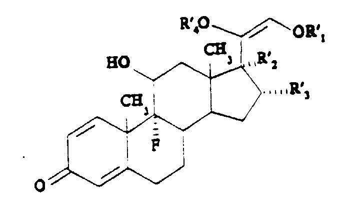 wherein:
R'₁ is selected from substituted or unsubstituted: C₁-C₅ alkyl, alkenyl, and alkynyl;
R'₂ is selected from hydrogen and substituted or unsubstituted: C₁-C₆ alkyl, alkenyl, alkynyl; acyloxy; and hydroxyl; wherein the substituent or substituents are selected from: C₁-C₃ alkyl; hydroxyl; and halogen;
R'₃ is selected from methyl and hydroxyl; and
R'₄ is selected from C₁-C₅ alkyl, alkenyl, and alkynyl.

10. The compound of claim 9, wherein:
R'₁ is selected from methyl, ethyl, and allyl;
R'₂ is selected from hydroxyl, acetyloxy, and propanoyloxy;
R'₃ is selected from methyl and hydroxyl; and
R'₄ is selected from methyl, ethyl, and allyl.

11. The compound of claim 10, wherein:
R'₁ is selected from methyl, ethyl, and allyl;
R'₂ is selected from hydroxyl, acetyloxy, and propanoyloxy; and
R'₃ is methyl.

12. An ophthalmic pharmaceutical composition comprising an anti-inflammatory effective amount of a compound of formula: 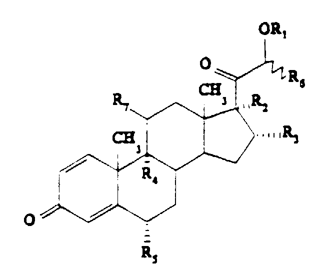 wherein:
R₁ is selected from substituted or unsubstituted: C₁-C₅ alkyl; C₃-C₆ (ring) and C₁-C₃ (alkyl) cycloalkyl and cycloalkylalkyl; aryl and arylalkyl, such as phenyl and benzyl; and C₂-C₅ alkenyl and alkynyl; wherein the substituent or substituents are selected from: C₁-C₆ alkoxy and alkyl; C₂-C₆ alkoxyalkyl, alkoxyalkoxyalkyl, alkylthioalkyl, hydroxyalkyl, aminoalkyl, nitriloalkyl; C₄-C₈ (beta-carboxyalkoxy)alkyl; and C₂-C₆ alkenoxy and alkynoxy;
R₂ is selected from hydrogen and substituted or unsubstituted: C₁-C₆ alkyl, alkenyl and alkynyl; acyloxy; and hydroxyl; wherein the substituent or substituents are selected from: C₁-C₃ alkyl; hydroxyl; and halogen (F, Cl, and Br);
R₃ is selected from methyl and hydroxyl;
R₄ is selected from hydrogen, fluoro, and chloro;
R₅ is selected from hydrogen, methyl, fluoro, and chloro;
R₆ is selected from hydrogen, methyl, ethyl, and allyl; and
R₇ is selected from hydroxyl, acetyloxy, propanoyloxy, and butanoyloxy; or a pharmaceutically acceptable salt thereof;
and an ophthalmically acceptable vehicle therefor.

13. The composition of claim 12, wherein:
R₁ is selected from methyl, ethyl, propyl, butyl, allyl, cyclopropyl, cyclobutyl, cyclopentyl, cyclohexyl, phenyl, benzyl, isopropyl, isobutyl, isoamyl, isovaleryl, 2-propynyl, and 2-methoxymethyl methylthiomethyl, hydroxyethyl, and methoxy ethoxymethyl;
R₂ is selected from hydrogen, methyl, ethyl, propyl, butyl, amyl, acetyloxy, propanoyloxy, butanoyloxy, valeroyloxy, 2-propynyl, and hydroxyl;
R₃ is selected from methyl and hydroxyl;
R₄ is selected from hydrogen, fluoro and chloro;
R₅ is selected from hydrogen, methyl, fluoro and chloro;
R₆ is selected from hydrogen, methyl, ethyl, and allyl; and
R₇ is hydroxyl.

14. The composition of claim 13, wherein R₂, R₃ and R₄ are, respectively, hydroxyl, methyl and fluoro.

15. The composition of claim 12, 13 or 14, wherein R₅ is methyl.

16. The composition of claim 14, wherein R₅ is hydrogen.

17. The composition of claim 13, wherein R₂ and R₃ are hydroxyl and R₄ and R₅ are fluoro.

18. The composition of claim 12, wherein R₂ is selected from hydrogen, acetyloxy, propanoyloxy, butanoyloxy, valeroyloxy and 2-propynyl.

19. The composition of claim 18, wherein R₃ is hydroxyl and R₄ and R₅ are fluoro.

20. The composition of claim 12, wherein R₁ is methyl, R₂ is hydroxyl, R₃ is methyl, R₄ is fluoro and R₅ is hydrogen.

21. An ophthalmic pharmaceutical composition comprising an anti-inflammatory effective amount of a compound of formula: 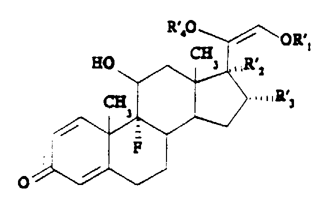 wherein:
R'₁ is selected from substituted or unsubstituted: C₁-C₅ alkyl, alkenyl, and alkynyl;
R'₂ is selected from hydrogen and substituted or unsubstituted: C₁-C₆ alkyl, alkenyl, alkynyl; acyloxy; and hydroxyl; wherein the substituent or substituents are selected from: C₁-C₃ alkyl; hydroxyl; and halogen;
R'₃ is selected from methyl and hydroxyl; and
R'₄ is selected from C₁-C₅ alkyl, alkenyl, and alkynyl;
and an ophthalmically acceptable vehicle therefor.

22. The composition according to claim 21, wherein:
R'₁ is selected from methyl, ethyl, and allyl;
R'₂ is selected from hydroxyl, acetyloxy and propanoyloxy;
R'₃ is selected from methyl and hydroxyl; and
R'₄ is selected from methyl, ethyl, and allyl.

23. The composition according to claim 22, wherein:
R'₁ is selected from methyl, ethyl, and allyl;
R'₂ is selected from hydroxyl, acetyloxy, and propanoyloxy; and
R'₃ is methyl.

24. A pharmaceutical composition containing a compound according to any of claims 1 to 11 and a pharmaceutically acceptable excipient.

25. Use of a compound according to any of claims 1 ot 11 for the preparation of a pharmaceutical composition for the treatment of ocular inflammation.

## Patentansprüche

1. Verbindung der Formel 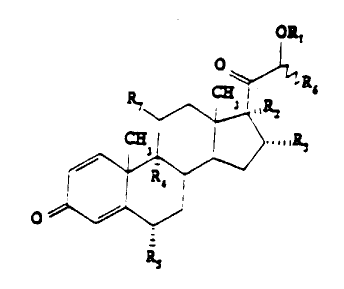 worin
R₁ ausgewählt ist aus substituierten oder unsubstituierten C₁-C₅-Alkyl-, C₃-C₆-(Ring) - und C₁-C₃-(Alkyl) - Cycloalkyl- und Cycloalkylalkyl-, Aryl- und Arylalkyl-, z.B. Phenyl- und Benzyl-, und C₂-C₅-Alkenyl-und Alkinylresten, wobei der Substituent oder die Substituenten ausgewählt sind aus C₁-C₆-Alkoxy- und Alkyl-resten, C₂-C₆-Alkoxyalkyl-, Alkoxyalkoxyalkyl-, Alkylthioalkyl-, Hydroxyalkyl-, Aminoalkyl-, Nitriloalkyl-, C₄-C₈- (beta-Carboxyalkoxy)alkyl- und C₂-C₆-Alkenoxy- und Alkinoxyresten;
R₂ ausgewählt ist aus Wasserstoff und substituierten oder unsubstituierten C₁-C₆-Alkyl-, Alkenyl- und Alkinylresten, Acyloxyresten und Hydroxylresten, wobei der Substituent oder die Substituenten ausgewählt sind aus C₁-C₃-Alkyl-, Hydroxyl- und Halogenresten (F, Cl und Br);
R₃ ausgewählt ist aus Methyl- und Hydroxylresten;
R₄ ausgewählt ist aus Wasserstoff, Fluor und Chlor;
R₅ ausgewählt ist aus Wasserstoff, Methylresten, Fluor und Chlor;
R₆ ausgewählt ist aus Wasserstoff, Methyl-, Ethyl- und Alylresten;
R₇ ausgewählt ist aus Hydroxyl-, Acetyloxy-, Propanoyloxy- und Butanoyloxyresten oder ein pharmazeutisch annehmbares Salz davon mit dem Vorbehalt, daß R₃ ein Hydroxylrest ist, wenn R₁ ein C₁-C₄-Alkylrest oder Methylthiomethylrest ist.

2. Verbindung nach Anspruch 1, worin
R₁ ausgewählt ist aus Methyl-, Ethyl-, Propyl-, Butyl-, Allyl-, Cyclopropyl-, Cyclobutyl-, Cyclopentyl-, Cyclohexyl-, Phenyl-, Benzyl-, Isopropyl-, Isobutyl-, Isoamyl-, Isovaleryl-, 2-Propinyl-, 2-Methoxymethyl-, Methylthiomethyl-, Hydroxyethyl- und Methoxyethoxymethylresten;
R₂ ausgewählt ist aus Wasserstoff, Methyl-, Ethyl-, Propyl-, Butyl-, Amyl-, Acetyloxy-, Propanoyloxy-, Butanoyloxy-, Valeroyloxy-, 2-Propinyl- und Hydroxylresten;
R₃ ausgewählt ist aus Methyl- und Hydroxylresten;
R₄ ausgewählt ist aus Wasserstoff, Fluor und Chlor;
R₅ ausgewählt ist aus Wasserstoff, Methylresten, Fluor und Chlor;
R₆ ausgewählt ist aus Wasserstoff, Methyl-, Ethyl- und Allylresten und
R₇ ein Hydroxylrest ist.

3. Verbindung nach Anspruch 2, worin R₂, R₃ und R₄ Hydroxyl-, Methyl-, bzw. Fluorreste sind.

4. Verbindung nach Anspruch 1, 2 oder 3, worin R₅ ein Methylrest ist.

5. Verbindung nach Anspruch 3, worin R₅ Wasserstoff ist.

6. Verbindung nach Anspruch 2, worin R₂ und R₃ Hydroxylreste sind und R₄ und R₅ Fluor ist.

7. Verbindung nach Anspruch 1, worin R₂ ausgewählt ist aus Wasserstoff, Acetyloxy-, Propanoyloxy-, Butanoyloxy-, Valeroyloxy- und 2-Propinylresten.

8. Verbindung nach Anspruch 7, worin R₃ ein Hydroxylrest ist und R₄ und R₅ Fluor sind.

9. Verbindung der Formel 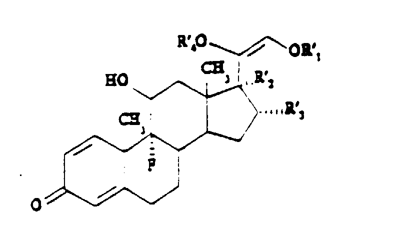 worin
R'₁ ausgewählt ist aus substituierten oder unsubstituierten C₁-C₅-Alkyl-, Alkenyl- und Alkinylresten;
R'₂ ausgewählt ist aus Wasserstoff und substituierten oder unsubstituierten C₁-C₆-Alkyl-, Alkenyl-, Alkinyl-, Acyloxy- und Hydroxylresten, worin der Substituent oder die Substituenten ausgewählt sind aus C₁-C₃-Alkyl-, Hydroxyl- und Halogenresten;
R'₃ ausgewählt ist aus Methyl- und Hydroxylresten und
R'₄ ausgewählt ist aus C₁-C₅-Alkyl-, Alkenyl- und Alkinylresten.

10. Verbindung nach Anspruch 9, worin
R'₁ ausgewählt ist aus Methyl-, Ethyl- und Allylresten;
R'₂ ausgewählt ist aus Hydroxyl-, Acetyloxy- und Propanoyloxyresten;
R'₃ ausgewählt ist aus Methyl- und Hydroxylresten und
R'₄ ausgewählt ist aus Methyl-, Ethyl- und Allylresten.

11. Verbindung nach Anspruch 10, worin
R'₁ ausgewählt ist aus Methyl-, Ethyl- und Allylresten;
R'₂ ausgewählt ist aus Hydroxyl-, Acetyloxy- und Propanoyloxyresten und
R'₃ ein Methylrest ist.

12. Ophthalmische pharmazeutische Zusammensetzung enthaltend eine entzündungshemmend wirksame Menge einer Verbindung der Formel 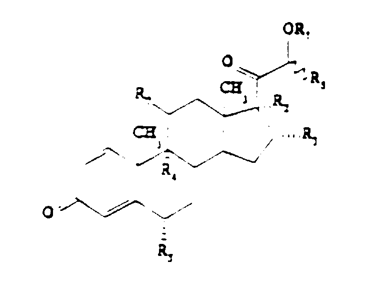 worin
R₁ ausgewählt ist aus substituierten oder unsubstituierten C₁-C₅-Alkyl-, C₃-C₆-(Ring)- und C₁-C₃-(Alkyl)-Cycloalkyl- und Cycloalkylalkyl-, Aryl- und Arylalkyl-, z.B. Phenyl- und Benzyl-, und C₂-C₅-Alkenyl-und Alkinylresten, wobei der Substituent oder die Substituenten ausgewählt sind aus C₁-C₆-Alkoxy- und Alkylresten, C₂-C₆-Alkoxyalkyl-, Alkoxyalkoxyalkyl-, Alkylthioalkyl-, Hydroxyalkyl-, Aminoalkyl-, Nitriloalkyl-, C₄-C₈-(beta-Carboxyalkoxy) alkyl- und C₂-C₆-Alkenoxy- und Alkinoxyresten;
R₂ ausgewählt ist aus Wasserstoff und substituierten oder unsubstituierten C₁-C₆-Alkyl-, Alkenyl- und Alkinylresten, Acyloxyresten und Hydroxylresten, wobei der Substituent oder die Substituenten ausgewählt sind aus C₁-C₃-Alkyl-, Hydroxyl- und Halogenresten (F, Cl und Br);
R₃ ausgewählt ist aus Methyl- und Hydroxylresten;
R₄ ausgewählt ist aus Wasserstoff, Fluor und Chlor;
R₅ ausgewählt ist aus Wasserstoff, Methylresten, Fluor und Chlor;
R₆ ausgewählt ist aus Wasserstoff, Methyl-, Ethyl- und Alylresten;
R₇ ausgewählt ist aus Hydroxyl-, Acetyloxy-, Propanoyloxy- und Butanoyloxyresten
oder ein pharmazeutisch annehmbares Salz davon und einen ophthalmisch annehmbaren Träger dafür.

13. Zusammensetzung nach Anspruch 12, worin
R₁ ausgewählt ist aus Methyl-, Ethyl-, Propyl-, Butyl-, Allyl-, Cyclopropyl-, Cyclobutyl-, Cyclopentyl-, Cyclohexyl-, Phenyl-, Benzyl-, Isopropyl-, Isobutyl-, Isoamyl-, Isovaleryl-, 2-Propinyl-, 2-Methoxymethyl-, Methylthiomethyl-, Hydroxyethyl- und Methoxyethoxymethylresten;
R₂ ausgewählt ist aus Wasserstoff Methyl-, Ethyl-, Propyl-, Butyl-, Amyl-, Acetyloxy-, Propanoyloxy-, Butanoyloxy-, Valeroyloxy-, 2-Propinyl- und Hydroxylresten;
R₃ ausgewählt ist aus Methyl- und Hydroxylresten;
R₄ ausgewählt ist aus Wasserstoff, Fluor und Chlor;
R₅ ausgewählt ist aus Wasserstoff, Methylresten, Fluor und Chlor;
R₆ ausgewählt ist aus Wasserstoff, Methyl-, Ethyl- und Allylresten und
R₇ ein Hydroxylrest ist.

14. Zusammensetzung nach Anspruch 13, worin R₂, R₃ und R₄ Hydroxyl-, Methyl- bzw. Fluorreste sind.

15. Zusammensetzung nach Anspruch 12, 13 oder 14, worin R₅ ein Methylrest ist.

16. Zusammensetzung nach Anspruch 14, worin R₅ Wasserstoff ist.

17. Zusammensetzung nach Anspruch 13, worin R₂ und R₃ Hydroxylreste sind und R₄ und R₅ Fluor sind.

18. Zusammensetzung nach Anspruch 12, worin R₂ ausgewählt ist aus Wasserstoff, Acetyloxy-, Propanoyloxy-, Butanoyloxy-, Valeroyloxy- und 2-Propinylresten.

19. Zusammensetzung nach Anspruch 18, worin R₃ ein Hydroxylrest ist und R₄ und R₅ Fluor sind.

20. Zusammensetzung nach Anspruch 12, worin R₁ ein Methylrest ist, R₂ ein Hydroxylrest ist, R₃ ein Methylrest ist, R₄ Fluor ist und R₅ Wasserstoff ist.

21. Ophthalmische pharmazeutische Zusammensetzung enthaltend eine entzündungshemmend wirksame Menge einer Verbindung der Formel 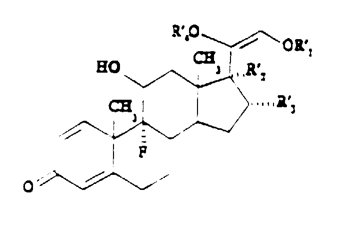 worin
R'₁ ausgewählt ist aus substituierten und unsubstituierten C₁-C₅-Alkyl-, Alkenyl- und Alkinylresten;
R'₂ ausgewählt ist aus Wasserstoff und substituierten oder unsubstituierten C₁-C₆-Alkyl-, Alkenyl-, Alkinyl-, Acyloxy- und Hydroxylresten, worin der Substituent oder die Substituenten ausgewählt sind aus C₁-C₃-Alkyl-, Hydroxyl- und Halogenresten;
R'₃ ausgewählt ist aus Methyl- und Hydroxylresten und
R'₄ ausgewählt ist aus C₁-C₅-Alkyl-, Alkenyl- und Alkinylresten und einen ophthalmisch annehmbaren Träger dafür.

22. Zusammensetzung nach Anspruch 21, worin
R'₁ ausgewählt ist aus Methyl-, Ethyl- und Allylresten;
R'₂ ausgewählt ist aus Hydroxyl-, Acetyloxy- und Propanoyloxyresten;
R'₃ ausgewählt ist aus Methyl- und Hydroxylresten und
R'₄ ausgewählt ist aus Methyl-, Ethyl- und Allylresten.

23. Zusammensetzung nach Anspruch 22, worin
R'₁ ausgewählt ist aus Methyl-, Ethyl- und Allylresten;
R'₂ ausgewählt ist aus Hydroxyl-, Acetyloxy- und Propanoyloxyresten und
R'₃ ein Methylrest ist.

24. Pharmazeutische Zusammensetzung enthaltend eine Verbindung nach einem der Ansprüche 1 bis 11 und einen pharmazeutisch annehmbaren Hilfsstoff.

25. Verwendung einer Verbindung nach einem der Ansprüche 1 bis 11 zur Herstellung einer pharmazeutischen Zusammensetzung zur Behandlung einer Augenentzündung.

## Revendications

1. Composé de la formule : 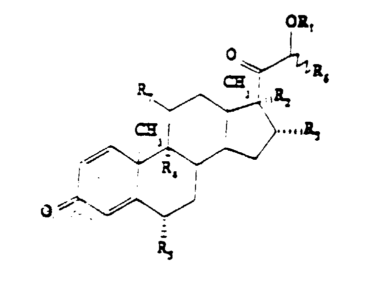 dans laquelle
R₁ est choisi parmi les radicaux qui suivent, substitués ou non substitués : alkyle en C₁ à C₅; C₃-C₆ (cycle) et (alkyl)C₁-C₃cycloalkyle et cycloalkylalkyle; aryle et arylalkyle, tel que phényle et benzyle; et alcynyle et alcényle en C₂ à C₅; où le substituant ou les substituants sont choisis parmi les radicaux qui suivent: alkyle et alcoxy en C₁ à C₆; nitriloalkyle, aminoalkyle, hydroxyalkyle, alkylthioalkyle, alcoxyalcoxyalkyle, alcoxyalkyle en C₂ à C₆; (bêta-carboxyalcoxy)-alkyle en C₄ à C₈; et alcynoxy et alcénoxy en C₂ à C₆;
R₂ est choisi parmi l'hydrogène et les radicaux qui suivent, substitués ou non substitués : alcynyle, alcényle, alkyle en C₁ à C₆; acyloxy; et hydroxyle; où le substituant ou les substituants sont choisis parmi les radicaux qui suivent : alkyle en C₁ à C₃; hydroxyle, et les atomes d'halogènes (F, Cl et Br);
R₃ est choisi parmi les radicaux méthyle et hydroxyle;
R₄ est choisi parmi l'hydrogène, le fluor et le chlore;
R₅ est choisi parmi l'hydrogène, le radical méthyle, le fluor et le chlore;
R₆ est choisi parmi l'hydrogène, les radicaux méthyle, éthyle et allyle; et
R₇ est choisi parmi les radicaux hydroxyle, acétyloxy, propanoyloxy et butanoyloxy;
ou un sel pharmaceutiquement acceptable de celui-ci, avec la conditions que R₃ représente le radical hydroxyle si R₁ représente un radical alkyle en C₁ à C₄ ou le radical méthylthiométhyle.

2. Composé suivant la revendication 1, caractérisé en ce que :
R₁ est choisi parmi les radicaux méthyle, éthyle, propyle, butyle, allyle, cyclopropyle, cyclobutyle, cyclopentyle, cyclohexyle, phényle, benzyle, isopropyle, isobutyle, isoamyle, isovaléryle, 2-propynyle, 2-méthoxyméthyle, méthylthiométhyle, hydroxyéthyle et méthoxyéthoxyméthyle.
R₂ est choisi parmi l'hydrogène et les radicaux qui suivent: méthyle, éthyle, propyle, butyle, amyle, acétyloxy, propanoyloxy, butanoyloxy, valéroyloxy, 2-propynyle et hydroxyle;
R₃ est choisi par les radicaux méthyle et hydroxyle;
R₄ est choisi parmi l'hydrogène, les atomes de fluor et de chlore;
R₅ est choisi parmi l'hydrogène, le radical méthyle, les atomes de fluor et de chlore;
R₆ est choisi parmi l'hydrogène, les radicaux méthyle, éthyle et allyle; et
R₇ est choisi parmi les radicaux hydroxyle.

3. Composés suivant la revendication 2, caractérisé en ce que R₂, R₃ et R₄ sont respectivement des radicaux hydroxyle, méthyle et du fluor.

4. Composé suivant la revendication 1, 2 ou 3, caractérisé en ce que R₅ représente le radical méthyle.

5. Composé suivant la revendication 3, caractérisé en ce que R₅ représente un atome d'hydrogène.

6. Composé suivant la revendication 2, caractérisé en ce que R₂ et R₃ représentent des radicaux hydroxyle et R₄ et R₅ sont des atomes de fluor.

7. Composé suivant la revendication 1, caractérisé en ce que R₂ est choisi parmi l'hydrogène, les radicaux acétyloxy, propanoyloxy, butanoyloxy, valéroyloxy et 2-propynyle.

8. Composé suivant la revendication 7, caractérisé en ce que R₃ représente le radical hydroxyle et R₄ et R₅ représentent des atomes de fluor.

9. Composé de la formule : 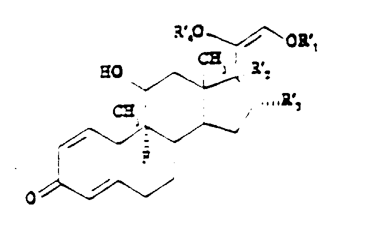 dans laquelle :
R'₁ est choisi parmi les radicaux qui suivent, substitués ou non substitués : alcynyle, alcényle et alkyle en C₁ à C₅;
R'₂ est choisi parmi l'hydrogène et les radicaux qui suivent, substitués ou non substitués : alcynyle, alcényle, alkyle en C₁ à C₆; acyloxy; et hydroxyle; où le substituant ou les substituants sont choisis parmi les radicaux alkyle en C₁ à C₃; hydroxyle; et les atomes d'halogènes;
R'₃ est choisi parmi les radicaux méthyle et hydroxyle; et
R'₄ est choisi parmi les radicaux alcynyle, alcényle et alkyle en C₁ à C₅.

10. Composé suivant la revendication 9, caractérisé en ce que :
R'₁ est choisi parmi les radicaux méthyle, éthyle et allyle;
R'₂ est choisi parmi les radicaux hydroxyle, acétyloxy et propanoyloxy;
R'₃ est choisi par les radicaux méthyle et hydroxyle; et
R'₄ est choisi parmi les radicaux méthyle, éthyle et allyle.

11. Composé suivant la revendication 10, caractérisé en ce que :
R'₁ est choisi parmi les radicaux méthyle, éthyle et allyle;
R'₂ est choisi parmi les radicaux hydroxyle, acétyloxy et propanoyloxy et
R'₃ représente le radical méthyle.

12. Composition pharmaceutique ophtalmique comprenant une quantité efficace anti-inflammatoire d'un composé de la formule : 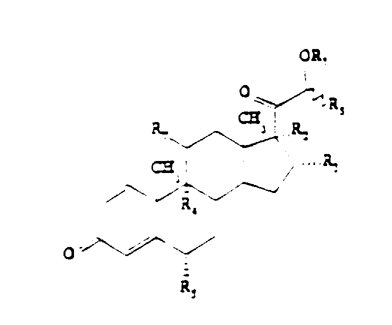 dans laquelle
R₁ est choisi parmi les radicaux qui suivent, substitués ou non substitués : alkyle en C₁ à C₅; C₃-C₆ (cycle) et (alkyl)C₁-C₃cycloalkyle et cycloalkylalkyle; aryle et arylalkyle, tel que phényle et benzyle; et alcynyle et alcényle en C₂ à C₅; où le substituant ou les substituants sont choisis parmi les radicaux qui suivent: alkyle et alcoxy en C₁ à C₆; nitriloalkyle, aminoalkyle, hydroxyalkyle, alkylthioalkyle, alcoxyalcoxyalkyle, alcoxyalkyle en C₂ à C₆; (bêta-carboxyalcoxy)-alkyle en C₄ à C₈; et alcynoxy et alcénoxy en C₂ à C₆;
R₂ est choisi parmi l'hydrogène et les radicaux qui suivent, substitués ou non substitués : alcynyle, alcényle, alkyle en C₁ à C₆; acyloxy; et hydroxyle; où le substituant ou les substituants sont choisis parmi les radicaux qui suivent : alkyle en C₁ à C₃; hydroxyle, et les atomes d'halogènes (F, Cl et Br);
R₃ est choisi parmi les radicaux méthyle et hydroxyle;
R₄ est choisi parmi l'hydrogène, le fluor et le chlore;
R₅ est choisi parmi l'hydrogène, le radical méthyle, le fluor et le chlore;
R₆ est choisi parmi l'hydrogène, les radicaux méthyle, éthyle et allyle; et
R₇ est choisi parmi les radicaux hydroxyle, acétyloxy, propanoyloxy et butanoyloxy; ou un sel pharmaceutiquement acceptable de ce composé
et un véhicule ophtalmiquement acceptable pour celui-ci.

13. Composition suivant la revendication 12, caractérisée en ce que:
R₁ est choisi parmi les radicaux méthyle, éthyle, propyle, butyle, allyle, cyclopropyle, cyclobutyle, cyclopentyle, cycohexyle, phényle, benzyle, isopropyle, isobutyle, isoamyle, isovaléryle, 2-propynyle et 2-méthoxyméthylthiométhyle, hydroxyéthyle et méthoxyéthoxyméthyle.
R₂ est choisi parmi l'hydrogène et les radicaux qui suivent: méthyle, éthyle, propyle, butyle, amyle, acétyloxy, propanoyloxy, butanoyloxy, valéroyloxy, 2-propynyle et hydroxyle;
R₃ est choisi par les radicaux méthyle et hydroxyle;
R₄ est choisi parmi l'hydrogène, les atomes de fluor et de chlore;
R₅ est choisi parmi l'hydrogène, le radical méthyle, les atomes de fluor et de chlore;
R₆ est choisi parmi l'hydrogène, les radicaux méthyle, éthyle et allyle; et
R₇ est choisi parmi les radicaux hydroxyle.

14. Composition suivant la revendication 13, caractérisée en ce que R₂, R₃ et R₄ représentent, respectivement, des radicaux hydroxyle, méthyle et du fluor.

15. Composition suivant les revendications 12, 13 ou 14, caractérisée en ce que R₅ représente le radical méthyle.

16. Composition suivant la revendication 14, caractérisée en ce que R₅ représente un atome d'hydrogène.

17. Composition suivant la revendication 13, caractérisée en ce que R₂ et R₃ sont des radicaux hydroxyle et R₄ et R₅ sont des atomes de fluor.

18. Composition suivant la revendication 12, caractérisée en ce que R₂ est choisi parmi l'hydrogène, les radicaux acétyloxy, propanoyloxy, butanoyloxy, valéroyloxy et 2-propynyle.

19. Composition suivant la revendication 18, caractérisée en ce que R₃ est le radical hydroxyle et R₄ et R₅ sont des atomes de fluor.

20. Composition suivant la revendication 12, caractérisée en ce que R₁ est le radical méthyle, R₂ est le radical hydroxyle, R₃ est le radical méthyle, R₄ est l'atome de fluor et R₅ représente l'hydrogène.

21. Composition pharmaceutique ophtalmique comprenant une quantité efficace anti-inflammatoire d'un composé de la formule : 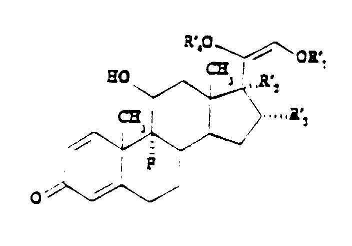 dans laquelle :
R'₁ est choisi parmi les radicaux qui suivent, substitués ou non substitués : alcynyle, alcényle et alkyle en C₁ à C₅;
R'₂ est choisi parmi l'hydrogène et les radicaux qui suivent, substitués ou non substitués : alcynyle, alcényle, alkyle en C₁ à C₆; acyloxy; et hydroxyle; où le substituant ou les substituants sont choisis parmi les radicaux alkyle en C₁ à C₃; hydroxyle; et les atomes d'halogènes;
R'₃ est choisi parmi les radicaux méthyle et hydroxyle; et
R'₄ est choisi parmi les radicaux alcynyle, alcényle et alkyle en C₁ à C₅;
et un véhicule ophtalmiquement acceptable pour celui-ci.

22. Composition suivant la revendication 21, caractérisée en ce que:
R'₁ est choisi parmi les radicaux méthyle, éthyle et allyle;
R'₂ est choisi parmi les radicaux hydroxyle, acétyloxy et propanoyloxy;
R'₃ est choisi par les radicaux méthyle et hydroxyle; et
R'₄ est choisi parmi les radicaux méthyle, éthyle et allyle.

23. Composition suivant la revendication 22, caractérisée en ce que:
R'₁ est choisi parmi les radicaux méthyle, éthyle et allyle;
R'₂ est choisi parmi les radicaux hydroxyle, acétyloxy et propanoyloxy et
R'₃ représente le radical méthyle.

24. Composition pharmaceutique contenant un composé suivant l'une quelconque des revendications 1 à 11 et un excipient pharmaceutiquement acceptable.

25. Utilisation d'un composé suivant l'une quelconque des revendications 1 à 11, pour la préparation d'une composition pharmaceutique destinée au traitement d'inflammations oculaires.
